Europäisches Patentamt

European Patent Office

Office européen des brevets

(11) Numéro de publication : **0 322 334 B1**

(12) **FASCICULE DE BREVET EUROPEEN**

(45) Date de publication du fascicule du brevet :
**26.02.92 Bulletin 92/09**

(51) Int. Cl.$^5$ : **A61F 5/02**

(21) Numéro de dépôt : **88480093.9**

(22) Date de dépôt : **08.12.88**

(54) **Prothèse inter-épineuse.**

(30) Priorité : **23.12.87 FR 8718210**

(43) Date de publication de la demande :
**28.06.89 Bulletin 89/26**

(45) Mention de la délivrance du brevet :
**26.02.92 Bulletin 92/09**

(84) Etats contractants désignés :
**AT BE CH DE ES FR GB GR IT LI LU NL SE**

(56) Documents cités :
**EP-A- 0 140 790**
**EP-A- 0 146 347**
**EP-A- 0 192 949**
**DE-A- 2 821 678**
**FR-A- 1 004 625**

(56) Documents cités :
**FR-A- 2 231 352**
**US-A- 4 255 820**
**US-A- 4 369 769**
**US-A- 4 604 995**
**US-A- 4 643 178**

(73) Titulaire : **CREMASCOLI FRANCE**
**111 Rue Léonard Arnaud**
**F-06700 Saint Laurent du Var (FR)**

(72) Inventeur : **Bronsard, Jean-Jacques**
**10 Impasse Wermert**
**13007 Marseille (FR)**

(74) Mandataire : **Hautier, Jean-Louis**
**OFFICE MEDITERRANEEN DE BREVETS**
**D'INVENTION 24 rue Masséna**
**F-06000 Nice (FR)**

EP 0 322 334 B1

## Description

L'invention a pour objet une prothèse inter-épineuse inter- vertébrale de suspension verrouillage et un ou plusieurs coussinets inter-épineux.

L'invention s'applique notamment au décambrage des vertèbres pour combattre une lordose.

Actuellement, on utilise des plaques métalliques, des tiges, (pour des scolioses), des cerclages (pour des athroses discales) ou autres moyens analogues, mais métalliques. Ces moyens nécessitent la mise en oeuvre de techniques lourdes. Ces techniques peuvent entrainer des dégâts anatomiques supplémentaires. Tous ces moyens permettent d'obtenir un blocage complet. Ce blocage complet peut avoir de nombreux inconvénients.

L'état de la technique est le suivant :

– US-A.4.643.178 : ce brevet décrit un lacet chirurgical et son procédé de mise en oeuvre. Il n'y a pas de cale souple faisant office de coussin.

– US-A.4.369.769 : ce brevet décrit un dispositif de fixation pour bloquer les vertèbres avec tous les inconvénients décrits plus hauts. Il se compose de plaques métalliques, de tiges filetées avec des manchons coulissants le long desdites tiges. Lesdits manchons servant de moyens de calage.

– US-A.4.604.995 : ce brevet décrit un implant chirurgical qui est utilisé pour apporter une stabilité à la colonne thoracique lombaire par la fixation d'un implant sur la colonne avec une instrumentation segmentaire de la colonne. L'implant comprend un bouton unitaire ayant une configuration généralement rectangulaire fermée par une paire de branches écartées, dupliquées de façon identique l'une sur l'autre (comme dans un miroir) et espacées de façon égale sur toute leur longueur.

Une pièce arrondie relie la paire de branches dans leur extrémité supérieure, tandis qu'une fermeture en barrière forme l'extrémité de la partie inférieure de la paire de branches, à l'exception d'une petite ouverture de la barrière. L'ouverture provoquée par la barrière facilite la fixation des fils sous-laminaires pour l'implantation à la colonne.

– EP 0 192 949 : ce brevet décrit une méthode pour fabriquer une prothèse extensible en tissu capable de supporter une charge, formant un certain nombre de boucles allongées autour d'au moins deux épingles espacées, à partir d'un filament continu de matériau câblé, rassemblant les terminaisons en boucle à une terminaison de boucle pour former un oeillet, y compris par exemple par fusion, de façon à empêcher les terminaisons en boucle de se séparer et, de préférence, tordre les boucles entre les terminaisons de la boucle pour assurer une distribution égale de la charge.

– FR-A-2.231.352 : ce brevet décrit un dispositif auxiliaire pour le traitement chirurgical de la malformation dite thorax en entonnoir suivant lequel on sépare une partie des côtes du thorax et l'on corrige leur position défectueuse par le moyen de brides de tension, caractérisé en ce qu'il comprend deux telles brides symétriques l'une de l'autre et dont les extrémités libres s'utilisent pour assurer chacune la fixation à une côte, un organe d'assemblage propre à relier rigidement les brides, et un élément destiné à fixer l'organe d'assemblage au sternum.

L'invention tend à résoudre tous ces inconvénients par l'utilisation de moyens de maintien souple. Ces moyens peuvent également permettre d'obtenir un blocage partiel.

A cet effet, le ligament selon l'invention est composé d'un lacet plat semi-élastique. Ledit lac et est pourvu à une extrémité d'un oeillet de transfilage.

Le lacet peut comporter un moyen de repérage radiologique tel qu'un filament radioopaque.

Le lacet peut avoir des longueurs différentes par exemple 20, 35, 50cm.

Selon un mode de realisation préféré, le lacet est tissé en fibre polyester. Sa résistance à la traction peut être environ de 54kg, et 30% d'élasticité à 50Kg.

Associés au lacet, on utilise des coussinets inter-épineux.

Ces coussinets sont fabriqués dans le même matériau que le lacet. Ces coussinets sont de petits cylindres creux. L'épaisseur d'un coussinet peut être d'environ 10mm. Ces coussinets peuvent être superposés en fonction de la hauteur de l'espace inter-épineux dans lesquels le lacet peut être passé. La structure du coussinet permet à la fois le maintien de l'espace inter-épineux à la hauteur voulue et le verrouillage par tassement en extension et conservation d'une relative mobilité dans les mouvements de flexion.

Le coussinet est réalisé dans la même matière que le lacet par exemple en polyester plein par tissage dans la masse. Le polyester peut être une fibre telle que le DACRON (marque commerciale déposée).

Les caractéristiques techniques du lacet et de ses coussinets seront mieux compris avec les dessins ci-joints donnés à titre d'exemples indicatifs et non limitatifs. Ils représentent un mode de réalisation préféré selon l'invention. Ils permettront de comprendre aisément l'invention.

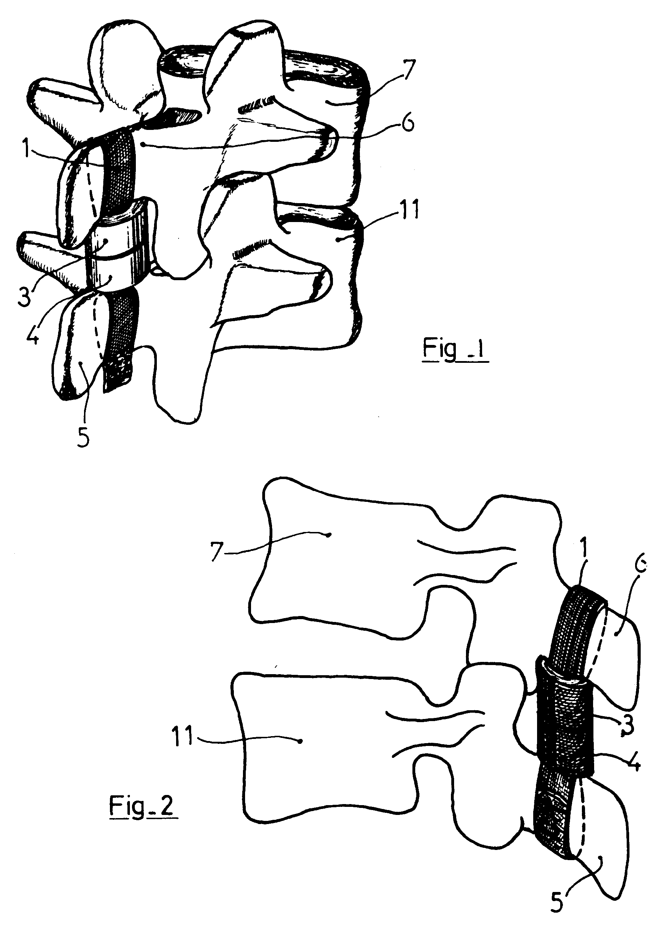

La figure 1 est une vue en perspective de deux vertèbres vues de 3/4 arrière et sur lesquelles sont mis en place un lacet et deux coussinets selon l'invention.

La figure 2 est une vue selon la figure 1 mais vue latéralement.

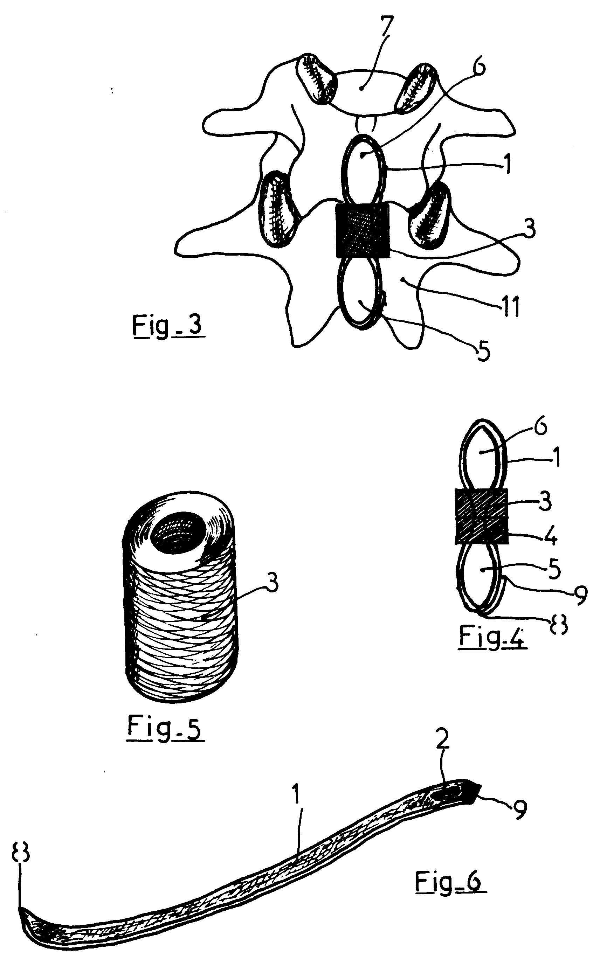

La figure 3 est une vue selon la figure 1 vue postérieure.

La figure 4 est une vue schématique vue en coupe des vertèbres vues à la figure 1, ladite coupe passant au niveau du lacet et des épineuses.

La figure 5 est une vue en perspective d'un coussinet.

La figure 6 est une vue en perspective d'un lacet.

Le lacet 1 est fabriqué dans une matière semi-élastique, il est plat et pourvu à une extrémité d'un oeillet de transfilage 2.

En combinaison avec ce lacet 1, il est utilisé des coussinets 3, 4. Le coussinet 3 ou 4 est réalisé dans le même matériau que le ligament. Il a la forme d'un cylindre creux.

Les interventions pour hernie discale semblent actuellement bien codifiées et donnent, selon les différentes statistiques, 80 à 85% de bons résultats, deux problèmes à long terme persistent :

1) La conservation de la hauteur discale (discopathie "Facett-Syndrom"- contact inter-épineux).

2) Les récidives en situ (la plupart des opérateurs s'accordant pour laisser en place le maximum de disque sain).

Une solution simple semble apporter une amélioration dans ce domaine : la mise en place d'un lacet de polyester semi-élastique inter-épineux 1 dont le but est :

a) le verrouillage intervertébral par laçage inter-épineux.

b) la suspension par l'adaptation d'un ou plusieurs coussinets inter-épineux semi-élastiques 3, 4 maintenant une hauteur suffisante aidant ainsi à la conservation de l'espace discal pendant le temps de la cicatrisation complète du disque et du ligament commun vertébral postérieur.

Une des extrémités du lacet 1 est passée à la base de l'épineuse 5 sous-jacente dans le ligament inter-épineux, (toujours plus gracile à ce niveau).

La tension s'effectue après avoir fait pénétrer une extrémité du lacet 1 dans l'oeillet terminal 2 de l'autre extrémité.

(La distance coussinet 3 ou 4 oeillet 2 doit toujours rester très courte).

Le réglage de la tension s'effectue très facilement grâce à cet artifice.

Le lacet 1 est suturé à lui-même, après l'oeillet 2 et avant l'oeillet 2, de façon à éviter les effets d'une éventuelle fragilisation du lacet 1 par l'oeillet.

Une petite partie du lacet 1 est ensuite rabattue vers la base des coussinets 3 ou 4 et suturée à ce niveau.

Le réglage de la tension du lacet et de l'efficacité des coussinets 3, 4 nécessite une table d'opération permettant les mouvements de lordose et de cyphose pendant l'intervention.

Des clichés dynamiques en flexion extension peuvent être réalisés en post-opératoire pour apprécier l'effet stabilisateur du procédé.

– Il s'agit d'une technique simple ;

– Ne nécessitant pas de dégâts anatomiques supplémentaires ;

– Assurant un verrouillage localisé ;

– Permettant, par la même, une rééducation précoce ;

– Inocuité : les effets bio-mécaniques, la tolérance, et le devenir à long terme ont été particulièrement bien étudiés.

L'originalité de cette technique utilisant des lacets 1 et des coussinets 3, 4 réside dans :

1°) Le type de malade auxquels elle s'adresse (hernies discales).

2°) L'absence de resection osseuse.

3°) L'interposition d'une prothèse inter-épineuse 2, 3, 4 semi-élastique assure le maintien de la hauteur discale et la stabilité intervertébrale en extension.

Les coussinets 3, 4 sont fabriqués dans le même matériau que le ligament.

Il s'agit de petits cylindres creux, d'une épaisseur d'environ 10mm. On peut superposer ces coussinets 3, 4 en fonction de la hauteur de l'espace inter-épineux dans lesquels le lacet 1 peut être passé. La structure permet à la fois un maintien de l'espace inter-épineux à la hauteur voulue, et un verrouillage par tassement en extension et conservation d'une relative mobilité dans les mouvements de flexion.

La technique d'utilisation du lacet 1 et d'un ou de plusieurs coussinets 3, 4 est la suivante.

Le lacet 1 est placé sur le bord de l'épineuse 6 de la vertèbre supérieure 7. Puis le lacet 1 est transfilé en double dans le ou les coussinets inter-épineux 3, 4. Ceux-ci sont mis en place, bien "calés" dans l'espace inter-épineux en s'aidant d'un passe-fil spécialement adapté à la manoeuvre.

Le nombre et la taille des coussinets 3, 4 utilisés dépend de l'espace discal que l'on veut maintenir. Une fois réglées, les deux extrémités 8, 9 du lacet sont passées sous l'épineuse 5 de la vertèbre inférieure 11. Le réglage de la tension s'effectue après avoir fait pénétrer l'extrémité 8 du lacet 1 dans l'oeillet 2 et après que les deux extrémités 8, 9 aient entouré l'épineuse 5. La suture du lacet 1 s'effectue comme indiqué plus haut.

## Revendications

1. Prothèse inter-épineuse de verrouillage et de suspension caractérisée par le fait qu'elle est constituée par un lacet plat semi-élastique (1) et un ou plusieurs coussinets (3, 4} fabriqués dans le même matériau que le lacet (1).

2. Prothèse selon la revendication 1 caractérisée par le fait que le lacet (1) est pourvu à une extrémité (9) d'un oeillet de transfilage.

3. Prothèse selon la revendication 1 caractérisée par le fait que le coussinet (3 ou 4) a la forme d'un petit cylindre creux.

4. Prothèse selon la revendication 1 caractérisée par le fait que le lacet est en polyester semi-élastique.

5. Prothèse selon la revendication 1 caractérisée par le fait que le coussinet (3 ou 4) est en polyester semi-élastique plein tissé dans la masse.

6. Prothèse selon la revendication 1 caractérisée par le fait que le coussinet (3 ou 4) peut avoir une épaisseur d'environ 10mm.

7. Prothèse selon l'une quelconque des revendications 1 ou 4 caractérisée par le fait qu'il comporte un filament opaque de manière à permettre le repérage radiologique.

**Claims**

1. A prothesis implanted between vertebral spinous processes for blockage and suspension, **characterized** in that it consists of a half-elastic flat band (1) and of one or several pads (3, 4) of the same material as the band (1).

2. The prothesis according to claim 1, characterized in that on one end (9) the band is provided with an eyelet for passing-through purposes.

3. The prothesis according to claim 1, characterized in that the pad (3 or 4) has the shape of a small hollow cylinder.

4. The prothesis according to claim 1, characterized in that the band is a half-elastic polyester.

5. The prothesis according to claim 1, characterized in that the pad (3 or 4) is of half-elastic polyester fully woven in its mass.

6. The prothesis according to claim 1, characterized in that the pad (3 or 4) may have a thickness of about 10 mm.

7. The prothesis according to one of claims 1 or 4, characterized in that it comprises a filament which is opaque to radiation to permit radiological location.

**Patentansprüche**

1. Zwischen Wirbeldornfortsätzen eingesetzte Prothese zur Verriegelung und Aufhängung, dadurch gekennzeichnet, daß sie aus einem halbelastischen Flachband (1) und einem oder mehreren Kissen (3, 4) aus dem gleichen Material wie das Band (1) besteht.

2. Prothese nach Anspruch 1, dadurch gekennzeichnet, daß das Band an einem Ende (9) mit einer Öse zum Durchziehen versehen ist.

3. Prothese nach Anspruch 1, dadurch gekennzeichnet, daß das Kissen (3 oder 4) die Form eines kleines Hohlzylinders hat.

4. Prothese nach Anspruch 1, dadurch gekennzeichnet, daß das Band aus halbelastischem Polyester ist.

5. Prothese nach Anspruch 1, dadurch gekennzeichnet, daß das Kissen (3 oder 4) aus halbelastischem vollständig in der Masse verwebtem Polyester ist.

6. Prothese nach Anspruch 1, dadurch gekennzeichnet, daß das Kissen (3 oder 4) eine Dicke von ungefähr 10 mm aufweisen kann.

7. Prothese nach einem der Ansprüche 1 oder 4, dadurch gekennzeichnet, daß sie einen strahlenundurchlässigen Faden aufweist, um eine radiologische Ortung zu ermöglichen.

Fig _1

Fig _2

Fig_3

Fig_5

Fig_4

Fig_6